# EUROPEAN PATENT SPECIFICATION

(11) **EP 2 176 269 B1**
(45) Date of publication and mention of the grant of the patent: **09.03.2011**
(21) Application number: 08773248.3
(22) Date of filing: 08.07.2008
(51) Int. Cl.: C07D 495/02

(54) **A METHOD OF MANUFACTURING 5-[2-CYCLOPROPYL-1-(2-FLUOROPHENYL)-2-OXOETHYL]-4,5,6,7- TETRAHYDROTHIENO[3,2-C]PYRIDIN-2-YL ACETATE (PRASUGREL)**
VERFAHREN ZUR HERSTELLUNG VON ESSIGSÄURE-5-[2-CYCLOPROPYL-1-(2-FLUORPHENYL)-2-OXOETHYL]-4,5,6,7-TETRAHYDROTHIENO[3,2-C]PYRIDIN-2-YL-ESTER (PRASUGREL)
PROCÉDÉ DE FABRICATION D'ACÉTATE DE 5-[2-CYCLOPROPYL-1-(2-FLUOROPHÉNYL)-2-OXOÉTHYL]-4,5,6,7-TÉTRAHYDROTHIÉNO[3,2-C]PYRIDIN-2-YLE (PRASUGREL)

(30) Priority: 09.07.2007 CZ 20070456
(43) Date of publication of application: 21.04.2010
(73) Proprietor: Zentiva, k.s., Dolni Mecholupy 102 37 Praha 10 (CZ)
(72) Inventor: STEPANKOVA, Hana, 282 01 Cesky Brod (CZ); HAJICEK, Josef, 128 00 Praha 2 (CZ)
(74) Representative: Jirotkova, Ivana
(86) International application number: PCT/CZ2008/000079
(87) International publication number: WO 2009/006859

(56) References cited:
- EP-A- 0 192 535
- EP-A- 0 542 411
- WO-A-2005/118548
- WO-A-2006/042481

## Description

### Technical Field

The invention deals with a new method of manufacturing the known substance reducing blood coagulation - prasugrel - of formula I.

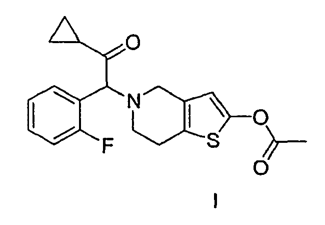

The chemical name of prasugrel is 5-[2-cyclopropyl-1-(2-fluorophenyl)-2-oxoethyl]-4,5,6,7-tetrahydrothieno[3,2-c]pyridin-2-yl acetate.

### Background Art

Prasugrel, a method of its preparation and its use as an anti-aggregation agent for patients having the risk of vein obstruction by a blood clot was first described in the patent no. EP 542411.

The manufacture of prasugrel in accordance with said patent can be summarized in Scheme 1.

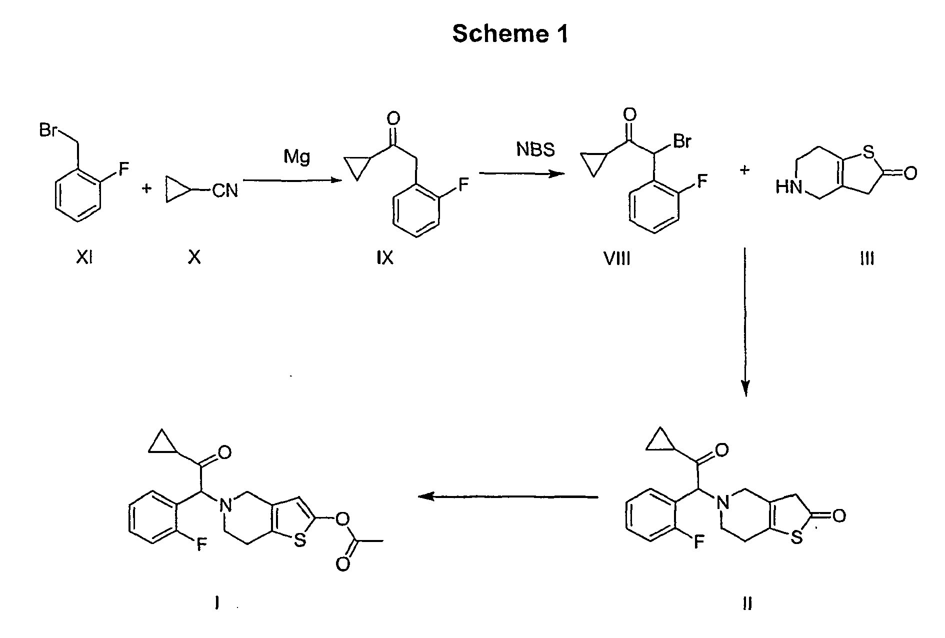

In accordance with the above mentioned document a Grignard reagent prepared from 2-fluorobenzylbromide (XI) reacts with cyclopropylcyanide (X) in ether and provides the compound (IX). The compound (IX) is brominated with bromine in CCl₄ or with N-bromosuccinimide (NBS) in the presence of dibenzoylperoxide⁵ to the bromo derivative (VIII), which is added to the nitrogen atom of the compound (III) in the presence of potash to give the compound (II). The compound (II) is converted to final prasugrel (I) by reaction with acetanhydride in the presence of NaH in DMF⁵.

A similar procedure can be inferred from the older document EP 192 535 and it is indicated here in Scheme 2.

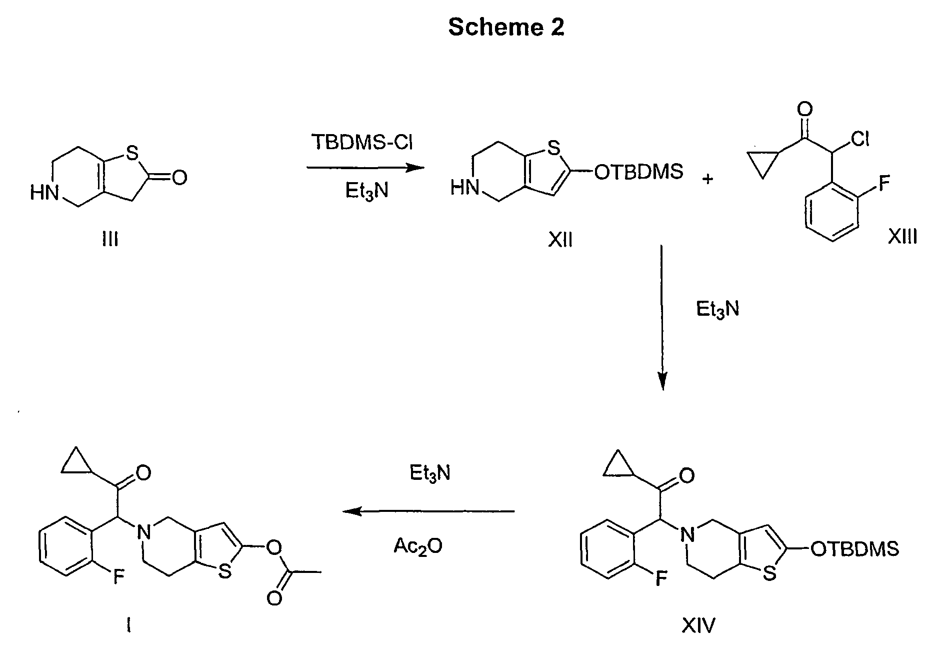

A reaction of thienopyridin-2-one (III) with tert-butyldimethylsilylchloride (TBDMS-Cl) in dichloromethane in the presence of triethylamine provides silylated enolether (XII), which reacts with the compound (XIII) again in the presence of triethylamine in dichloromethane to give the compound (XIV). The final prasugrel of formula I is then prepared from the substance (XIV) first after deprotection of Et₃N and DMAP and subsequent acetylation with acetanhydride.

Besides α-haloketones (VIII) and (XIII), another key intermediate is 2-oxo-thienotetrahydropyridine (III), which is used in the hydrochloride form in Scheme 1 and in the tosylate form in Scheme 2. Its preparation has been published by Sanofi⁴ and starts from commercially available 4,5,6,7-tetrahydrothieno[3,2,-c]pyridine (XX); see Scheme 3.

First, the nitrogen atom (96%) is blocked by reaction with triphenylmethylchloride in dichloromethane in the presence of Et₃N and the protected compound (XIX) is prepared. This compound (XIX) is converted to the lithium salt (XVIII), which provides the derivative (XVII) by reaction with tri-n-butylborate, which derivative (XVII) is oxidized with 30% hydrogen peroxide in-situ to the compound (XVI), which is immediately hydrolyzed to tritylated thienopyridone (XV) (64 %). This reaction step is carried out in a mixture of THF and hexane at the temperatures of -40 °C to -20 °C. In the last step the trityl group is deprotected with 98% formic acid (90 °C, 1 hour) (81%) and the desired compound (III) is produced.

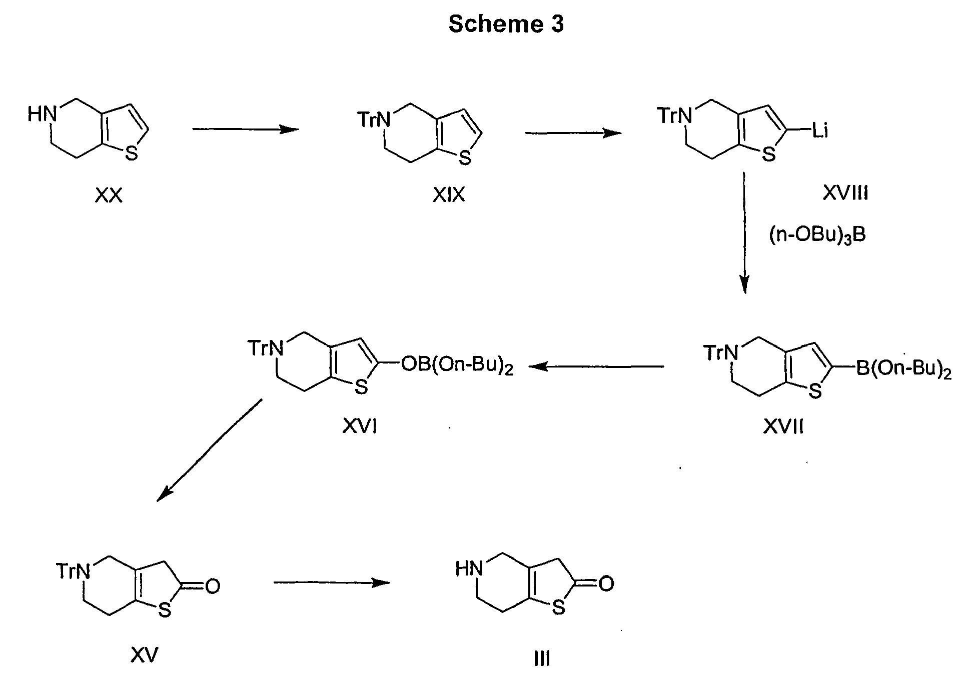

In comparison to the known methods the manufacturing method of the present invention provides the possibility of using a cheaper input raw material and avoiding problematic steps in the preparation of α-haloketones.

### Disclosure of Invention

The object of the invention is a new method of manufacturing 5-[2-cyclopropyl-1-(2-fluorophenyl)-2-oxoethyl]-4,5,6,7-tetrahydrothieno[3,2-c]pyridin-2-yl acetate, known under the INN name prasugrel, of formula I.

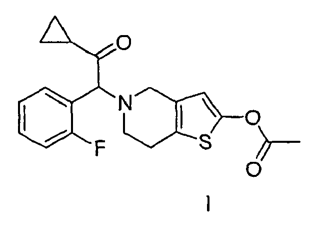

The starting substance of formula VI 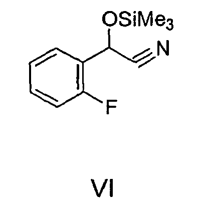 is reacted with a cyclopropyl magnesium halide to produce the substance of formula V, 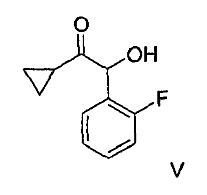 which is further reacted with methanesulfonyl chloride to generate the methanesulfonate of formula IV 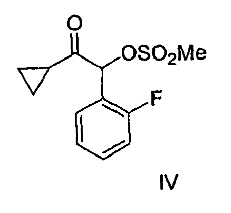 which is further reacted with the compound of formula III 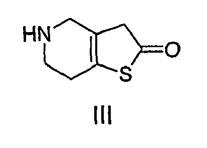 to be converted to the substance of formula II 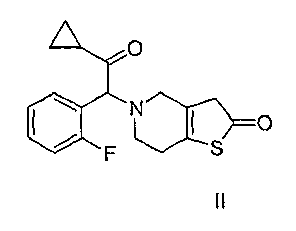 and the latter is then converted to the substance of formula I with an acetylation reagent.

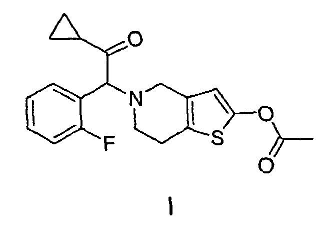

The invention also relates to the preparation and use of the key intermediate 3-cyclopropyl-1-(2-fluorophenyl)-3-oxopropyl methanesulfonate of formula IV for the preparation of prasugrel of formula I.

The key step of the whole process is the conversion of the compound of formula IV to the amide of formula II by reaction with 2-oxo-thienotetrahydropyridine of formula III.

### Detailed description of the invention

The synthesis of the invention can be briefly described by the following scheme.

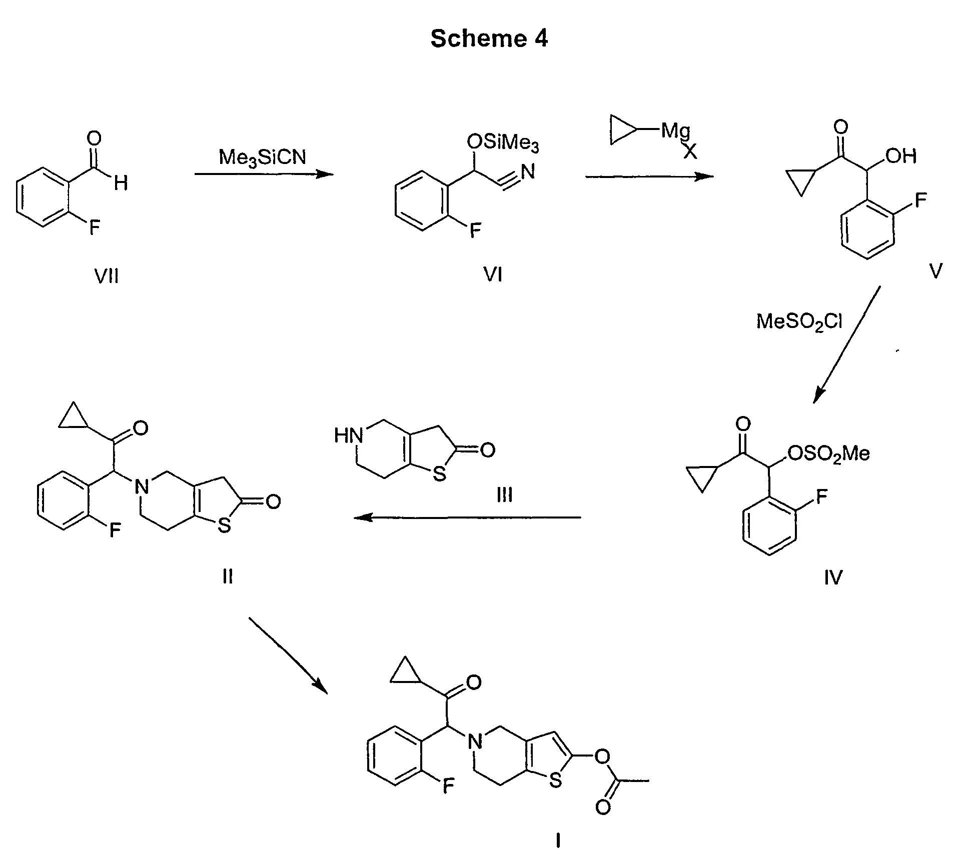

The invention relates to the preparation of prasugrel by a procedure using 3-cyclopropyl-1-(2-fluorophenyl)-3-oxopropyl methanesulfonate (IV) for alkylation of 2-oxo-thienotetrahydro-pyridine (III). Managing this stage makes it possible to use a cheaper starting material such as o-fluoro-benzaldehyde and to avoid problematic halogenation leading to α-haloketones.

The reaction occurs in aprotic solvents of the type of dimethylformamide, dimethylsulfoxide, acetonitrile, tetrahydrofuran or chlorinated aliphatic or aromatic hydrocarbons at a temperatures of 10 to 150 °C, preferably at the boiling point of the solvent used. The reaction occurs in the presence of a base, which is used in the molar proportion with regard to methanesulfonate (IV) of 1 : 1 to 3 : 1. Alkaline hydroxides or carbonates, or alkylamines, can be used as the bases. Bases with good solubility in the reaction environment are preferably selected. Amines have also proved to be useful, e.g. trialkylamines such as triethylamine. The reaction is further supported by sources of halides such as a tetraalkyl ammonium halide, e.g. bromide, or lithium iodide. A substance that is soluble in the reaction mixture is more advantageous, i.e. rather the ammonium salt. The amount of added halides varies in the molar proportions to the starting mesylate of 1:1 to 3 : 2.

Processing of the starting o-fluorobenzaldehyde (VII) is further enabled by its reaction with trimethyl silylcyanide in the presence of zinc iodide, producing the silylated nitrile of 2-fluoromandelic acid (VI). The reaction proceeds at a reduced temperature from -10 to +10 °C in aprotic solvents.

The next step is the Grignard reaction proceeding conventionally in dried ether and removal of the protecting silyl group to obtain the substance V.

After introduction of a well-leaving group such as methanesulfonate or toluenesulfonate to give the substance IV into the resulting α-hydroxy ketone (V) the latter is used for the above mentioned reaction with 2-oxo-thienotetrahydro-pyridine (III) to give the substance II.

The acetylation reaction leading to the final product, prasugrel (I), is carried out in an aprotic solvent in the presence of a strong base, e.g. sodium hydride. The reaction proceeds at a reduced temperature and with use of an acetylation agent such as acetanhydride or acetylchloride.

### Examples

### Preparation of the compound of formula VI

### Example 1

2.0 g (16.11 mmol) of 2-fluorobenzaldehyde were dissolved in 20 ml of dichloromethane. While being stirred the solution was cooled to a temperature in the range of -5 to +0 °C. A catalytic quantity of anhydrous zinc iodide was added to this solution and 1.76 g (17.72 mmol) of trimethylsilyl cyanide was added dropwise at the temperature of 0 to +3 °C during 45 minutes. The cooling bath was put aside and the reaction mixture was stirred at the room temperature for 18 hours. After this period the reaction mixture was decomposed with 15 ml of water. The dichloromethane fraction was separated, dried with sodium sulfate and concentrated in a rotational vacuum evaporator to dryness. The crude product was then chromatographed on silica gel in the petroleum ether: ethyl acetate 5 : 2 system. 3.1 grams of the compound of formula VI were obtained as colourless oil (86.1 %).

¹H NMR (250 MHz, CDCl3) δ(ppm): 7.67 (ddd, *J* = 15.0, 7.4, 1.9 Hz, 1H), 7.42 (m, 1H), 7.26 (ddd, *J* = 15.2, 7.6, 1.1 Hz, 1H), 7.12 (ddd, *J* = 10.3, 8.2, 1.1 Hz, 1H), 5.80 (s, 1H), 0.27 (s, 9H);
¹³C NMR (250 MHz, CDCl3) δ(ppm):159.4 (d, *J_{CF}* = 248.9 Hz), 131.3 (d, *J_{CF}* = 8.4 Hz), 128.4 (d, *J_{CF}* = 2.6 Hz), 124.7 (d, *J_{CF}* = 3.6 Hz), 123.8 (d, *J_{CF}* = 13.2 Hz), 118.3, 115.6 (d, *J_{CF}* = 20.7 Hz), 57.6 (d, *J_{CF}* = 5.3 Hz), 0.49

### Preparation of the compound of formula V

### Example 2

In a three-neck flask equipped with a magnetic stirrer, thermometer, dropping funnel and an inert gas inlet, 4.4 g of magnesium metal, 200 mg of iodine and 150 g of ether, which was dried by distillation with sodium before the reaction, were charged. A solution of 15 g (0.123 mol) of cyclopropylbromide in 50 ml of dried ether was added dropwise to this mixture during spontaneous reflux for 1.5 hours. The resulting reaction mixture was then stirred at room temperature for another 2 hours. Then, a solution of 13.0 g (58.25 mmol) of the compound of formula VI in a mixture of 30 ml of ether and 50 ml of tetrahydrofuran was slowly added to the resulting Grignard reagent during 1.5 hours; the temperature of the reaction mixture was maintained between 22 and 28 °C with moderate cooling. After the addition of all the solution the reaction mixture was stirred at the room temperature for 18 hours. After this period the reaction mixture was cooled in a water + ice bath to the internal temperature of +5 to +10 °C and carefully decomposed with 150 ml of 2N HCl. The resulting mixture was stirred at the room temperature for 5.5 hours, then diluted with 100 ml of ether. The organic fraction was separated, the aqueous fraction was again extracted with 100 ml of ether. The combined organic fractions were washed with 100 ml of water, 100 ml of a saturated NaCl solution, dried with anhydrous sodium sulfate and concentrated in a rotational vacuum evaporator to dryness. 10.3 g of the crude product was obtained in this manner, which was chromatographed on silica gel using of the petroleum ether: ether 5 : 1 eluent. 5.95 g (52,6 %) of the compound of formula V were obtained as a colourless oil.

¹H NMR (250 MHz, CDCl3) δ(ppm): 7.31 (m, 2H), 7.14 (m, 2H), 5.59 (s, 1H), 4.36 (s, 1H), 1.90 (m, 1H), 1.18 (m, 1H), 1.02 (m, 2H), 0.84 (m, 1H);
¹³C NMR (250 MHz, CDCl3) δ(ppm): 208.8, 160.6 (d, *J_{CF}* = 253,0 Hz), 130.3 (d, *J_{CF}* = 8.6 Hz), 129.1 (d, *J_{CF} =* 3.7 Hz), 125.7 (d, *J_{CF} =* 13.7 Hz), 124.7 (d, *J_{CF}* = 3.6 Hz), 115.8 (d, *J_{CF}* = 21.7 Hz), 73.6 (d, *J_{CF}* = 3.3 Hz), 17.2 (d, *J_{CF}* = 3.2 Hz), 12.3 (d, *J_{CF} =* 7.1 Hz)

### Preparation of the compound of formula IV

### Example 3

1.5 g (7.73 mmol) of the compound of formula V from the preceding example were dissolved in 50 ml of dichloromethane, 1.95 g (19.35 mol) of triethylamine were added to the solution and while being stirred the reaction mixture was cooled to the temperature of 0 to +2 °C. At this temperature 2.13 g (19.32 mmol) of methanesulfonyl chloride was added dropwise to the reaction mixture. The reaction mixture was stirred at the temperature of 0 to +2°C for 1.25 hours and then decomposed by addition of 25 ml of water. The dichloromethane fraction was separated and washed with 25 ml of 1 N HCl, 25 ml of water and dried with anhydrous sodium sulfate. The crude product was then chromatographed on silica gel with the mixture of petroleum ether: ethyl acetate 5 :2.
1.79 g (85 %) of the compound of formula IV was obtained as white crystalline substance with the melting temperature of 48-51 °C.

¹H NMR (250 MHz, CDCl3) δ (ppm): 7.41 (m, 2H), 7.20 (m, 2H), 6.39 (s, 1H), 3.10 (m, 3H), 2.02 (m, 1H), 1.08 (m, 4H)

¹³C NMR (250 MHz, CDCl3) δ(ppm): 202.4, 160.4 (d, *J_{CF}* = 250,3 Hz), 132.0 (d, *J_{CF}* = 8.3 Hz), 130.0 (d, *J_{CF}* = 2.8 Hz), 125.0 (d, *J_{CF}* = 3.7 Hz), 120.6 (d, *J_{CF}* = 14.2 Hz), 116.2 (d, *J_{CF}* = 21.1 Hz), 79.5 (d, *J_{CF}* = 2.8 Hz), 39.3, 17.8 (d, *J_{CF}* = 1.4 Hz); 12.5 (d, *J_{CF}* = 8.3 Hz)

### Preparation of the compound of formula II

### Example 4

0.695 g (2.55 mmol) of the compound of formula IV were dissolved in 20 ml of acetone, which was previously dried with anhydrous sodium sulfate. At the room temperature 425 mg (3.19 mmol) of lithium iodide were added to the resulting solution. The resulting reaction mixture was stirred at the room temperature for 1 hour. The undissolved fraction was then filtered through fritted glass; the filtration cake was washed with acetone. The filtrate was concentrated in a rotational vacuum evaporator to dryness. The evaporation residue was dissolved in 13 ml of dichloromethane and added to the solution prepared from 1.0 g (3.06 mmol) of the compound of formula III, 0.75 ml of triethylamine and 10 ml of dichloromethane. The reaction mixture was stirred at the room temperature for 2.5 hours. Then, the reaction mixture was diluted with 10 ml of water. The dichloromethane fraction was separated, dried with anhydrous sodium sulfate and concentrated in a rotational vacuum evaporator to dryness. The crude product was chromatographed on silica gel; eluent - toluene : ethyl acetate 3:1. 200 mg of the compound of formula II were obtained.

¹H NMR (250 MHz, CDCl3) δ(ppm): 7.25 (m, 4H), 6.03 (dt, *J* = 5.5, 1.5 Hz, 1H), 4.85 (d, *J* = 6.8 Hz, 1H), 4.09 (ddd, *J* = 12.5, 6.0, 1.6 Hz, 1H), 3.93 (ddd, *J* = 23.4, 11.7, 1.9 Hz, 1H), 3.1 (m, 2H), 2.85 (d, *J* = 12.2 Hz, 1H), 2.53 (ddd, *J* = 24.5, 12.2, 1.9 Hz, 1H), 2.10 (m, 1H), 1.91 (ddd, *J* = 25.4, 12.6, 4.1 Hz, 1H), 1.05 (m, 2H), 0.86 (m, 2H);

### Example 5

0.608 g of the compound of formula III (1.85 mmol) were stirred in 20 ml of dichloromethane; 0.373 g of triethylamine (3.7 mmol) were added to the mixture. After formation of a clear solution 353 mg (1.68 mmol) of tetramethyl ammonium bromide and 0.46 g of the compound of formula IV from Example 3 (1.68 mmol) were added to the mixture. The resulting reaction mixture was heated up to reflux for 20 hours. Then, it was cooled to the room temperature and extracted with 2 x 5 ml of water. The organic fraction was separated, dried with anhydrous sodium sulfate and concentrated in a rotational vacuum evaporator to dryness. The crude product was chromatographed on silica gel with the toluene : ethyl acetate 3 : 1 solvent mixture. In this manner 364 mg of the compound of formula II was prepared in the form of a honey-like substance that contained toluene residues.

NMR: the same as in the case of the compound of Example 4.

### Preparation of 5-[2-cyclopropyl-1-(2-fluorophenyl)-2-oxoethyl]-4,5,6,7-tetrahydrothieno-[3,2-c]pyridin-2-yl acetate (compound of formula I)

### Example 6

364 mg of the compound of formula II were dissolved in 1.27 ml of dimethylformamide and 0.75 ml of acetanhydride in an inert atmosphere. The solution was cooled in a water + ice bath to the temperature of 0 to +5 °C and 156 ml of a 60% dispersion of NaH in mineral oil were added to the solution in parts. The reaction mixture was first stirred being cooled to 0 to +5 °C for 30 minutes and then at the room temperature for 3.5 hours. After this period the reaction mixture was diluted with 10 ml of ethyl acetate and carefully decomposed by addition of 3 ml of water. The organic layer was separated, washed with 5 ml of a saturated solution of NaCl, dried with anhydrous sodium sulfate. After concentrating in a rotational vacuum evaporator the crude product was chromatographed on silica gel with the toluene : ethyl acetate 3 : 1 solvent mixture. 324 mg of an oily product was obtained, which was crystallized from 2 ml of diethylether.
120 mg of the compound of formula I with the melting temperature of 120.5-124.6 °C were obtained.

¹H NMR (250 MHz, CDCl3) δ(ppm): 7.47 (ddd, *J* = 14.7, 7.4, 1.7 Hz, 1H), 7.31 (m, 1H), 7.14 (m, 2H), 6.26 (s, 1H), 4.82 (s, 1H), 3.51 (m, 2H), 2.89 (m, 1H), 2.79 (m, 3H), 4.30 (m, 1H), 2.25 (s, 3H), 1.03 (m, 2H), 0.85 (m, 2H); ¹³C NMR (250 MHz, CDCl3) δ(ppm): 207.7, 167.7, 161.3 (d, *J_{CF} =* 247,6 Hz), 149.5, 130.6 (d, *J_{CF}* = 3.5 Hz), 129.9 (d, *J_{CF}* = 8.4 Hz), 129.4, 125.8, 124.4 (d, *J_{CF}* = 3.5 Hz), 122.1 (d, *J_{CF} =* 14.1 Hz), 115.8 (d, *J_{CF}* = 22.9 Hz), 112.0, 71.6, 50.5, 48.4, 25.0, 20.6,18.3,12.0, 11.4;

## Claims

1. A method of manufacturing 5-[2-cyclopropyl-1-(2-fluorophenyl)-2-oxoethyl]-4,5,6,7-tetrahydrothieno[3,2-c]pyridin-2-yl acetate, designated with the INN name prasugrel, of formula I 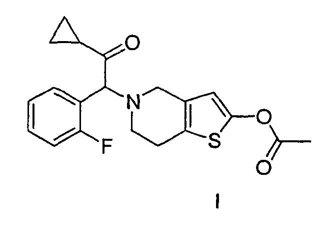 **characterized in that** the substance of formula VI 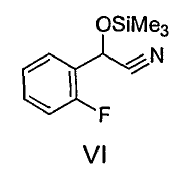 is reacted with a cyclopropyl magnesium halide to produce the substance of formula V, 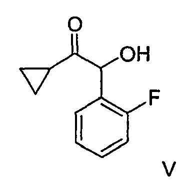 which is further reacted with methanesulfonyl chloride to give the methanesulfonate of formula IV 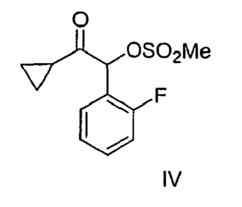 which is further reacted with the compound of formula III 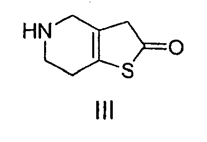 to be converted to the substance of formula II 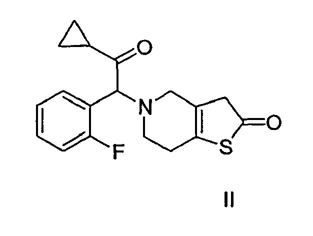 and the latter is converted to the substance of formula I with an acetylation agent.

2. A method of manufacturing 5-[2-cyclopropyl-1-(2-fluorophenyl)-2-oxoethyl]-5,6,7,7a-tetrahydrothieno[3,2-c]pyridin-2(4H)-on of formula (II), **characterized in that** the methanesulfonate of formula IV is converted to the corresponding amide by reaction with the substance of formula III or its salt.

3. The method according to claim 2, **characterized in that** the reaction is carried out in the presence of a tetraalkyl ammonium bromide.

4. The method according to claim 2 or 3, **characterized in that** the reaction is carried out in an aprotic solvent at a temperature of 10 to 150 °C.

5. The method according to claim 4, **characterized in that** an aprotic solvent is used whose boiling temperature is in the range of 10 to 150 °C.

6. The method according to any of claims 3-5, **characterized in that** the reaction is carried out for 1 to 120 hours.

7. The method according to any of claims 3-6, **characterized in that** before purification the resulting product is converted to the crystalline base.

8. The method according to any of claims 3-6, **characterized in that** the oily product is subject to distillation.

9. The method according to any of claims 3-6, **characterized in that** the product is purified by the chromatographic method.

10. 1-Cyclopropyl-3-(2-fluorophenyl)-3-hydroxypropan-1-one of formula V

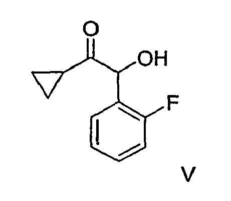

11. 3-Cyclopropyl-1-(2-fluorophenyl)-3-oxopropyl methanesulfonate of formula IV

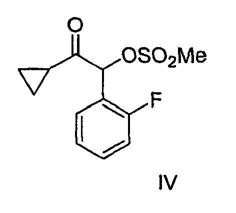

## Patentansprüche

1. Verfahren zur Herstellung von 5-[2-Cyclopropyl-1-(2 fluorophenyl)-2-oxoethyl]-4,5,6,7-tetrahydrothieno[3,2-c]pyridin-2-ylacetat, mit dem INN-Namen Prasugrel bezeichnet, mit der Formel I 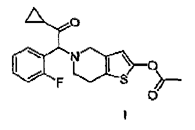 **dadurch gekennzeichnet, dass** die Substanz der Formel VI 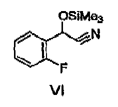 mit einem Cyclopropylmagnesiumhalogenid reagiert wird, zur Erzeugung der Substanz der Formel V 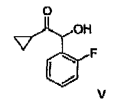 die weiterhin mit Methansulfonylchlorid reagiert wird, unter Erhalt des Methansulfonates der Formel IV 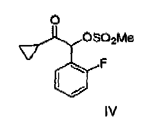 das weiter mit der Verbindung der Formel III reagiert wird 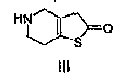 zum Umwandeln in die Substanz der Formel II 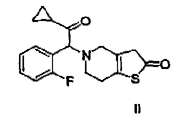 und die zuletzt Genannte in die Substanz der Formel I mit einem Acetylierungsmittel umgewandelt wird.

2. Verfahren zur Herstellung von 5-[2-Cyclopropyl-1-(2-fluorophenyl)-2-oxoethyl]-5,6,7,7a-tetrahydrothieno[3,2-c]pyridin-2(4H)-on der Formel (II), **dadurch gekennzeichnet, dass** das Methansulfonat der Formel IV in das entsprechende Amid durch Reaktion mit der Substanz der Formel III oder dessen Salz umgewandelt wird.

3. Verfahren nach Anspruch 2, **dadurch gekennzeichnet, dass** die Reaktion in der Gegenwart eines Tetraalkylammoniumbromides durchgeführt wird.

4. Verfahren nach Anspruch 2 oder 3, **dadurch gekennzeichnet, dass** die Reaktion in einem aprotischen Lösungsmittel bei einer Temperatur von 10 bis 150°C durchgeführt wird.

5. Verfahren nach Anspruch 4, **dadurch gekennzeichnet, dass** ein aprotisches Lösungsmittel verwendet wird, dessen Siedetemperatur im Bereich von 10 bis 150°C liegt.

6. Verfahren nach einem der Ansprüche 3 bis 5, **dadurch gekennzeichnet, dass** die Reaktion für 1 bis 120 Stunden durchgeführt wird.

7. Verfahren nach einem der Ansprüche 3 bis 6, **dadurch gekennzeichnet, dass** vor der Reinigung das resultierende Produkt in die kristalline Base umgewandelt wird.

8. Verfahren nach einem der Ansprüche 3 bis 6, **dadurch gekennzeichnet, dass** das ölige Produkt einer Destillation unterworfen wird.

9. Verfahren nach einem der Ansprüche 3 bis 6, **dadurch gekennzeichnet, dass** das Produkt durch das chromatographische Verfahren gereinigt wird.

10. 1-Cyclopropyl-3-(2-fluorophenyl)-3-hydroxypropan-1-on der Formel V

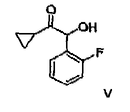

11. 3-Cyclopropyl-1-(2-fluorophenyl)-3-oxopropylmethan-sulfonat der Formel IV

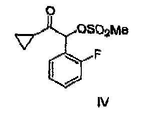

## Revendications

1. Un procédé de fabrication de 5-[2-cyclopropyl-1-(2-fluorophenyl)-2-oxoéthyl]-4,5,6,7-tétrahydrothiéno[3,2-c]pyridin-2-yl acétate, désigné dans le système INN par le terme de «prasugrel», présentant la formule I 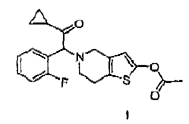 **caractérisé en ce que** la substance de formule VI 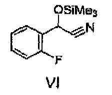 réagit avec un halogénure de cyclopropyl magnésium pour produire la substance de formule V, 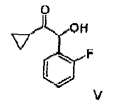 que l'on fait ensuite réagir avec le chlorure de methanesulfonyl pour produire le méthanesulfonate de formule IV 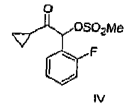 que l'on fait en outre réagir avec le composé de formule III 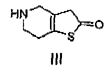 que l'on convertit en la substance de formule II 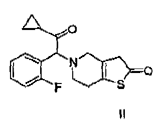 et laquelle est convertie en la substance de formula I par action d'un agent d'acétylation.

2. Un procédé de fabrication de 5-[2-cyclopropyl-1-(2-fluorophényl)-2-oxoéthyl]-5.6.7.7a-tétrahydrothieno-[3.2-c]pyridin-2(4H)-one de formule (II), **caractérisé en ce que** le méthanesulfonate de formule IV est converti en l'amide correspondante par réaction avec la substance de formule III ou un de ses sels.

3. Le procédé selon la revendication 2, **caractérisé en ce que** la réaction est effectuée en présence de bromure de tétraalkyl ammonium.

4. Le procédé selon la revendication 2 ou 3, **caractérisé en ce que** la réaction est effectuée dans un solvant aprotique à une température de 10 à 150°C.

5. Le procédé selon la revendication 4, **caractérisé en ce que** est employé un solvant aprotique dont la température d'ébullition est de l'ordre de 10 à 150°C.

6. Le procédé selon une quelconque des revendications 3 à 5, **caractérisé en ce que** la réaction est effectuée en une période de 1 à 120 heures.

7. Le procédé selon une quelconque des revendications 3 à 6, **caractérisé en ce que**, avant purification, le produit résultant est converti en base cristalline.

8. Le procédé selon une quelconque des revendications 3 à 6, **caractérisé en ce que** le produit huileux est soumis à une distillation.

9. Le procédé selon une quelconque des revendications 3 à 6, **caractérisé en ce que** le produit est purifié par chromatographie.

10. 1-Cyclopropyl-3-(2-fluorophényl)-3-hydroxypropan-1-one de formule V

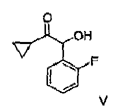

11. 3-Cyclopropyl-1-(2-fluorophényl)-3-oxopropyl méthanesulfonate de formule IV
